Europäisches Patentamt

European Patent Office

Office européen des brevets

(11) Veröffentlichungsnummer: **0 327 935 B1**

# EUROPÄISCHE PATENTSCHRIFT

(12)

(45) Veröffentlichungstag der Patentschrift: **23.12.92**

(51) Int. Cl.⁵: **A47L 9/02**

(21) Anmeldenummer: **89101682.6**

(22) Anmeldetag: **01.02.89**

---

(54) Vorrichtung zur Entkeimung von Räumen und Bodenbelägen.

---

(30) Priorität: **09.02.88 DE 3803825**

(43) Veröffentlichungstag der Anmeldung:
**16.08.89 Patentblatt 89/33**

(45) Bekanntmachung des Hinweises auf die
Patenterteilung:
**23.12.92 Patentblatt 92/52**

(84) Benannte Vertragsstaaten:
**AT BE CH DE ES FR GB GR IT LI LU NL SE**

(56) Entgegenhaltungen:
**GB-A- 648 967**
**GB-A- 2 179 962**
**US-A- 2 590 152**

(73) Patentinhaber: **Interlava AG
Contrada di Sassello 2
CH-6900 Lugano 1(CH)**

(72) Erfinder: **Kurz, Gerhard
Bruckenäcker 11
W-7000 Stuttgart 80(DE)**

(74) Vertreter: **Otte, Peter, Dipl.-Ing.
Tiroler Strasse 15
W-7250 Leonberg(DE)**

## Beschreibung

Stand der Technik

Die Erfindung geht aus von einem Staubsauger nach dem Oberbegriff des Anspruchs 1.

Ein bekannter Staubsauger dieser Art (US-A-2 590 152) umfaßt einen einstückigen Klopfsauger, der auf Rädern über die zu reinigende Fläche geführt ist und in seinem vorderen Teil eine Saugdüse ausbildet, in welcher eine gleichzeitig von dem Saugturbinenantrieb in eine Drehbewegung versetzte Klopfbürste rotiert. Nach hinten angrenzend an die Klopfbürste ist in einem eigenen Gehäuse ein UV-Strahler angeordnet, der gleichzeitig durch ein Abdeckgitter die zu reinigende Fläche bestrahlt und an welchem zusätzlich die angesaugte, staubbeladene Luft zur Entkeimung auch vorbeigeführt ist, durch Prallbleche allerdings so gesteuert wird, daß ein Zusetzen des UV-Strahlers mit Staubpartikeln verhindert wird.

Um zu verhindern, daß der UV-Strahler bei einem Anheben des Staubsaugers bei der Bedienungsperson gesundheitliche Schäden hervorruft, sind Schalter in zwei Ausführungsformen vorgesehen, die in diesem Fall den UV-Strahler abschalten.

Die erste Ausführungsform umfaßt einen Quecksilberschalter, der bei einer auf das Staubsaugergehäuse einschließlich der Düse ausgeübten Kippbewegung durch das dann von den Kontakten wegfließende Quecksilber den Stromkreis zum UV-Strahler öffnet und die Abgabe von Strahlung unterbricht. Das bedeutet allerdings, daß eine Unterbrechung des Stromkreises nur dann möglich ist, wenn das Staubsaugergehäuse in der richtigen Richtung verkantet wird, da der Quecksilberschalter in der anderen Kippposition die beiden Kontakte nur umso stärker überbrückt.

Bei einer anderen Ausführungsform ist ein zusätzliches Laufrad vorgesehen, welches unter Federspannung auf die zu reinigende Fläche gedrückt wird; bei einem Verkippen des Staubsaugers hebt dieses Laufrad ab und öffnet hierdurch gleichzeitig über eine entsprechende Hebelwirkung einen Mikroschalter.

Um hier beide Kipprichtungen zu umfassen, müßten eigentlich zwei Laufräder vorgesehen sein, da andernfalls auch diese Einrichtung nur bei einem vollständigen Abheben des schwergewichtigen Geräts vom Boden ansprechen kann.

Problematisch ist allerdings, daß gerade bei einem Stillstand des Geräts, wenn dieses also nicht von einer Bedienungsperson über die zu reinigende Fläche geführt wird, keine Abschaltmöglichkeiten vorgesehen sind, obwohl dies der häufigste Fall der Arbeitsunterbrechung bei Staubsaugern ist. Dem UV-Strahler wird in diesem Fall Gelegenheit gegeben, seine schädliche Strahlung konzentriert auf eine sehr schmale Fläche auszuüben und entsprechend schädliche Wirkungen zu entfalten, was schon bei Stillständen in der Größenordnung von Minutenbruchteilen zu einem Einbrennen der Strahlung und entsprechenden Beschädigungen an Teppichen, Mobiliar u.dgl. führen kann.

Ferner ist es bei einem ähnlichen einstückigen Klopfsauger (GB-A-648 967) bekannt, sowohl im Abluftstrom als auch nach vorn UV-Strahler nach Art von Scheinwerfern anzuordnen, wobei aber auch unterhalb des Staubsaugergehäuses oder ebenfalls scheinwerferartig nach rückwärts gerichtet UV-Strahler angeordnet werden können, die die zu bearbeitende Fläche bestrahlen. Zur Vermeidung von Beschädigungen durch den brennenden UV-Strahler ist ein Schalter vorgesehen, der vom Griffarm des Staubsaugers betätigt wird. Befindet sich der Griffarm in einer etwa senkrechten Ruheposition, dann wird der UV-Strahler abgeschaltet. Diese Abschaltung kann auch mit Zeitverzögerung erfolgen.

UV-Strahler bei Staubsaugern oder sonstigen Reinigungsgeräten sind deshalb vorgesehen, um Wohnräume von biologischen Schadstoffen, also Keimen und anderen Mikroorganismen, Viren und Bakterien, aber auch größeren Organismen wie die bekannte und gefährliche Hausstaubmilbe zu reinigen. In diesem Zusammenhang ist es ferner zusätzlich zu der Möglichkeit, in Klimaanlagen oder Luftbefeuchtern mit einer entkeimenden Strahlung zu arbeiten, auch bekannt, die von Staubsaugern erzeugte Abluft einer keimtötenden Ultraviolettstrahlung zu unterwerfen (DE-OS 29 10 104; DE-OS 30 09 365). Die Behandlung der Abluft kann dabei in einer Schleuse mit der Ultraviolettstrahlung erfolgen oder ein UV-Entkeimer ist als Nachfiltersatz für die Abluft so ausgebildet, daß eine Anzahl von UV-Strahlern längsgerichtet um einen Kanal angeordnet sind, der mit dem Luftauslaßstutzen verbunden ist (DE-OS 30 09 365).

Bei diesen die Abluft eines Staubsaugers entkeimenden Ausführungsformen ist bedenklich, daß hierdurch lediglich die Folgen einer biologischen Verseuchung von Räumen bekämpft wird, nämlich die Mikroorganismen, Feinstäube, Bakterien und Viren, die vom Staubsauger aufgenommen werden, während der eigentliche Entstehungsort, also beispielsweise Fußbodenbeläge jeglicher Art, Teppiche, Gardinen, Sessel u.dgl. unbehandelt bleiben. Ferner erscheint problematisch, daß die Abluft mit erheblicher Geschwindigkeit die keimtötende Zone passiert und daher nicht unbedingt sichergestellt sein muß, daß auch sämtliche Keime und sonstigen biologischen Schadstoffe einer hinreichenden Dosis an keimtötender Wirkung ausgesetzt werden. Falls dies nicht möglich ist, führt die Staubsaugerbehandlung nur zu einer stärkeren Verwirbelung biologischer Schadstoffe und kann das Übel nicht an der Wurzel packen.

Schließlich ist kürzlich ein spezielles chemisches Mittel für die Bekämpfung der Hausstaubmilbe auf den Markt gekommen (s. Zeitschrift highTech 2/88, S. 63), mit welchem Teppiche u.dgl. eingesprüht werden können. Dieses Mittel hilft aber bewußt nur gegen die Hausstaubmilbe und deren unangenehme Folge, beispielsweise allergische Belastung von Personen, die unter dieser Milbe und deren Ausscheidungen leiden. Es erscheint fraglich, ob man auf diese Weise eine wirksame Bekämpfung erzielen kann, weil dies ein sehr sorgfältiges Einnebeln sämtlicher Räume erforderlich macht, und, da es notwendigerweise auf chemischen Grundstoffen basiert, die Umwelt mit weiteren chemischen Erzeugnissen belastet.

Der Erfindung liegt die Aufgabe zugrunde, hier Abhilfe zu schaffen und, wenn auch über einen längeren Zeitraum, so doch wirksam sicherzustellen, daß die keimtötende Wirkung einer UV-Strahlung bzw. eines UV-Entkeimers wirksam und unmittelbar am Entstehungsort realisiert wird, und gleichzeitig zu verhindern, daß der UV-Strahler durch seine Strahlung eine negative Wirkung auf das behandelte Material ausüben kann.

Dabei ist es für sich gesehen bei einem Bügeleisen schon bekannt (GB-A-2 179 962), einen Bewegungs-Quecksilberschalter im Handgriff des Bügeleisens so anzuordnen, daß dieser in der Lage ist festzustellen, ob das Bügeleisen bewegt wird bzw. in welcher Position (aufgestellt) es sich befindet. Dementsprechend wird die Heizwicklung des Bügeleisens nach einer vorgegebenen Zeitdauer abgeschaltet.

Vorteile der Erfindung

Die Erfindung löst diese Aufgabe mit den kennzeichnenden Merkmalen des Anspruchs 1 und hat den Vorteil, daß eine einwandfreie keimtötende Wirkung der UV-Strahlung genau dort eingesetzt werden kann, wo die Saugdüse des Staubsaugers sich bewegt und wo auch bei sehr schnellem Arbeiten, also schnellem Hin- und Herschieben der Saugdüse der jeweils bestrahlte Bereich mit Sicherheit einer hinreichend langen Einwirkung der keimtötenden Strahlung ausgesetzt werden kann.

Zeichnung

Ein Ausführungsbeispiel der Erfindung ist in der Zeichnung schematisiert dargestellt und wird in der nachfolgenden Beschreibung näher erläutert. Es zeigen:

Fig. 1  eine mögliche Ausführungsform einer Saugdüse eines Staubsaugers in einer Ansicht von unten, stark schematisiert mit ergänzendem Abteil und die Aufnahme eines UV-Entkeimungsstrahlers und

Fig. 2  einen Schnitt durch die Bodensaugdüse der Fig. 1 mit teilweise weggelassenen Teilen.

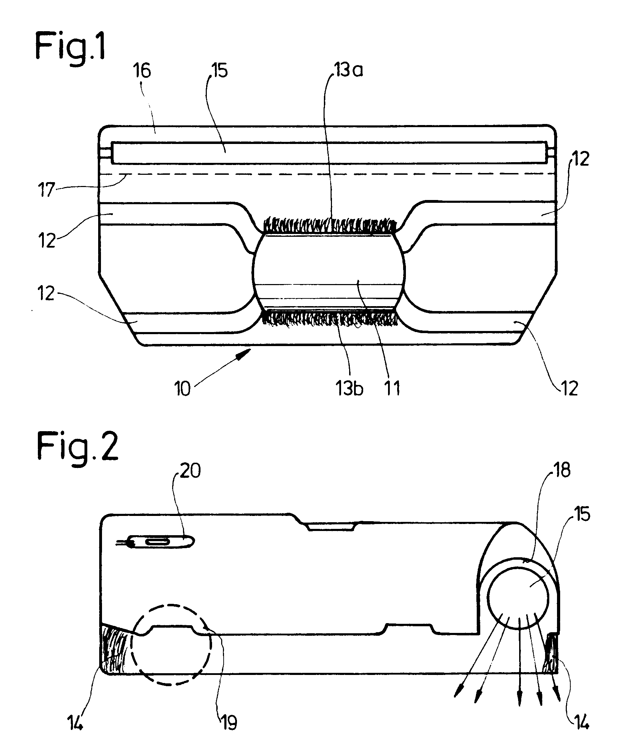

In der Darstellung der Fig. 1 ist die Staubsauger-Saugdüse mit 10 bezeichnet; es handelt sich hier bevorzugt um eine Saugdüse oder Bodendüse, die sich um einen Saugeinlaß 11 gruppierende, vertiefte Saugkanäle 12 aufweist, in im Grunde beliebiger Anordnung, da dies für die Erfindung keine Rolle spielt; dabei können um den Saugeinsatz selbst noch vorne und hinten (jeweils in der Arbeitsverschieberichtung gesehen) Bürstengruppen 13a, 13b) angeordnet sein.

Eine solche Bodensaugdüse für einen Staubsauger kann auch auf Rollen laufen, und sie kann einen in Fig. 1 nicht dargestellten, in Fig. 2 jedoch angedeuteten, beim Bearbeiten von Glattböden nach unten ausfahrbaren Rahmen 14 von bürstenartiger Struktur aufweisen.

An geeigneter Stelle und vorzugsweise in Querrichtung zur eigentlichen Arbeitsrichtung oder Verschieberichtung der Bürste befindet sich dann ein entsprechend länglich bauender UV-Strahler 15, der eine UV-Strahlung mit entsprechend starker keimtötender Wirkung abgibt. Dabei erstreckt sich die Strahlungsbreite dann, entsprechend der Länge der UV-Strahlerröhre, praktisch über die Breite der Saugbürste, so daß, wenn man die üblichen Überschneidungen beim Arbeiten einbezieht, stets die gesamte Fläche, die der Reinigungswirkung durch die Saugdüse 10 unterworfen wird, auch gleichzeitig der keimtötenden Strahlungswirkung des UV-Strahlers 15 unterworfen wird.

Der UV-Strahler 15 kann sich in einem eigenen, nach unten in Richtung auf das zu bearbeitende Material natürlich offenen zusätzlichen Abteil 16 der Saugdüse 10 befinden und von diesem geschützt umgeben sein, wobei die Einhöhe des UV-Strahlers 15 soweit von der unteren Bodenabschlußfläche der Saugdüse entfernt ist, daß Beschädigungen ausgeschlossen sind; es ist auch möglich, das Abteil 16 durch eine zusätzliche transparente Abdeckung gegen Fremdeinwirkung zu schützen. Dabei kann das Abteil 16 auch durch eine zusätzliche Trennwand 17 gegenüber dem restlichen Saugdüsenbereich abgetrennt sein. Es versteht sich, daß, um die eigentliche Reinigungswirkung nicht zu behindern, das Abteil 16 mit von diesem aufgenommenen UV-Strahler auch etwas erhöht mit Bezug auf die zu reinigende Bodenfläche oder die Bodenbeläge ausgebildet sein kann; ferner ist es zu empfehlen, entsprechend der Darstellung der Fig. 2 hinter der UV-Strahlerröhre 15, also in der Darstellung der Fig. 2 oberhalb und falls gewünscht herabgezogen auf beiden Seiten, ein reflektierendes Material 18 anzuordnen, welches beispielsweise auch eine halbschalenartige Kunststoffassung mit entsprechender reflektierender Beschichtung, ein Aluminiumreflektor o. dgl. sein kann. Hierdurch wird sichergestellt, daß die gesamte, von dem UV-Strahler 15 ausgehende Strahlung, zusätzlich zu dem direkt auffallenden Licht, auf das zu reinigende Material reflektiert und in seiner keimtötenden Wirkung gut ausgenutzt wird.

Es versteht sich, daß ein solcher UV-Strahler mit keimtötender Wirkung auch bei anderen Staubsaugerdüsen, beispielsweise Saugklopfdüsen, Möbeldüsen, Gardinendüsen u. dgl. in geeigneter Weise ausgebildet und eingesetzt werden kann, so daß überall dort, wo sich eine Reinigungswirkung entfaltet, wo also im Grunde mit Stäuben und insofern auch mit in Zusammenhang mit Verunreinigungen und Stäuben auftretenden biologischen Schadstoffen gerechnet werden muß, die keimtötende Wirkung einsetzt und dann stets wiederholend bei jeder Reinigung mit dem Staubsauger wieder realisiert wird.

Dabei ist in der auch den UV-Strahler 15 aufnehmenden Saugdüse ein Bewegungssensor angeordnet, der den UV-Strahler dann ausschaltet, wenn für eine vorgegebene Zeitdauer, beispielsweise, um hier einen numerischen, die Erfindung aber nicht einschränkenden Wert zu nennen, 3 Sekunden, eine Bewegung nicht stattfindet. Auf diese Weise wird verhindert, daß der UV-Strahler durch seine Strahlung eine negative Wirkung auf das behandelte Material ausüben kann.

Ein solcher Bewegungssensor kann bei einer Bodendüse beispielsweise mit den auf dem Boden gleitenden und sich drehenden Rädchen der Bodendüse gekoppelt sein, etwa als induktiver oder optischer, gegebenenfalls auch kapazitiver Sensor, der aus der Umdrehung des Rädchens über einen Geber die Bewegung der Saugdüse feststellt; in Fig. 2 ist ein solches, beim Schieben der Saugdüse über dem Boden sich drehendes Rad mit 19 bezeichnet. Eine weitere Möglichkeit, einen Bewegungssensor vorzusehen, besteht in der Anordnung eines Quecksilbergebers 20, der in Form eines länglichen Glasröhrchens mit zwei Kontakten auf der einen Seite und einer sich im Glasröhrchen frei bewegenden Quecksilberkugel ausgebildet ist. Durch das ständige Hin- und Herbewegen der Saugdüse bewegt sich auch das Quecksilber innerhalb des Quecksilbergebers 20 und schließt wiederholt Kontakte, so daß beispielsweise ein Monoflop mit einer vorgebbaren Einstelldauer jeweils in seinen instabilen Zustand durch die Kontaktgabe getriggert wird. Dies ist deshalb möglich, weil die Quecksilbermasse im Quecksilbergeber selbst als träge Masse reagiert und jeweils bei den Umkehrpunkten der Druck- und Zugbewegungen, der die Saugdüse beim Arbeiten ausgesetzt ist, im Röhrchen des Quecksilbergebers jeweils vorne und hinten anschlägt und dabei mindestens einmal die Kontakte schließt. Es können natürlich auch auf beiden Seiten Kontakte jeweils angeordnet werden. Das ständige Schwingen der Quecksilbermasse wird daher als Anzeige für die Bewegung der Bodendüse ausgenutzt.

Läuft der Monoflop ab, erfolgt also innerhalb vorgegebener Zeiten keine Rücktriggerung, dann ist dies ein Hinweis darauf, daß die Saugdüse nicht bewegt wird, und der Monoflop schaltet dann über eine geeignete Ansteuerschaltung, beispielsweise ein zwischengeschaltetes Relais o. dgl., den UV-Strahler 15 aus. Sobald der Monoflop wieder getriggert wird, erscheint an seinem anderen Ausgang hochliegendes Signal, und das Relais wird wieder zur Ansteuerung des UV-Strahlers 15 freigegeben. Hier sind beliebige Arten von Bewegungssensoren denkbar, die nicht alle angegeben zu werden brauchen.

**Patentansprüche**

1. Saugdüse (10) für einen Staubsauger in Form einer Bodendüse, Saugklopfdüse, Möbeldüse, Gardinendüse, zur Entkeimung von Räumen beliebiger Art sowie der in den Räumen befindlichen Einrichtungsgegenständen, Gardinen, Bodenbeläge, Teppichen o.dgl., mit mindestens einem, eine Ultraviolettstrahlung von keimtötender Wirkung erzeugenden Strahler (15) und einem den Strahler nach einer vorgegebenen Zeitdauer abschaltenden Sensor (20), wobei die von dem UV-Strahler (15) ausgehende Strahlung auf die von der Saugdüse (10) jeweils bearbeitete, sich in ihrer Position verändernde Fläche gerichtet ist, dadurch gekennzeichnet, daß der Sensor (20) als auf die Drehung eines Laufrades beim Betrieb der Saugdüse ansprechender oder als die Form eines bei Bewegung der Saugdüse ständige Kontaktgaben erzeugenden Quecksilberschalters aufweisender Bewegungssensor ausgebildet und in der Saugdüse eingebaut ist und vom Bewegungssensor ein Kippglied durch wiederholte Triggerung angesteuert wird, welches den UV-Strahler (15) so lange im eingeschalteten Zustand hält, bis bei Ausbleiben einer erneuten, auf eine direkte Bewegung der Saugdüse zurückgehenden Triggerung die Standzeit des Kippgliedes abläuft und der UV-Strahler abgeschaltet wird.

2. Saugdüse nach Anspruch 1, dadurch gekennzeichnet, daß der UV-Strahler (15) eine längliche Röhre ist, die quer zur normalen Arbeitsbewegungsrichtung der Saugdüse des Staubsaugers und die Breite der Saugdüse dabei ausfüllend, angeordnet ist.

3. Saugdüse nach Anspruch 1 oder 2, dadurch gekennzeichnet, daß der UV-Strahler (15) in einem zum Restdüsenbereich abgetrennten Abteil (16) angeordnet ist.

4. Saugdüse nach Anspruch 3, dadurch gekennzeichnet, daß das Abteil (16) durch eine gegenüber der keimtötenden UV-Strahlung durchlässigen Schutzabdeckung abgedeckt ist.

5. Saugdüse nach einem der Ansprüche 1 bis 4, dadurch gekennzeichnet, daß der zu behandelnden Fläche gegenüber und hinter bzw. seitlich zur Röhre des UV-Strahlers (15) angeordnet ein reflektierendes Material vorgesehen ist, welches die gesamte, von dem UV-Strahler (15) ausgehende Strahlung auf die zu behandelnde Fläche reflektiert.

6. Saugdüse nach Anspruch 5, dadurch gekennzeichnet, daß das reflektierende Material eine beschichtete, nach unten auf die zu behandelnde Fläche gerichtet offene, gegebenenfalls eine parabolische Form aufweisende Halbschale mit reflektierender Innenbeschichtung ist, die den UV-Strahler aufnimmt.

**Claims**

1. A suction nozzle (10) for a vacuum cleaner, which is in the form of a floor nozzle, suction beating nozzle, furniture nozzle, curtain nozzle, which is for sterilising rooms of any type as well as the furniture, curtains, floor coverings, carpets or the like located in the rooms, and which has at least one radiator (15) - generating ultraviolet radiation with germ-destroying action - and a sensor (20) switching off the radiator after a predetermined period of time, the radiation issuing from the UV radiator (15) being directed onto the surface which is being treated by the suction nozzle (10) in this instance and which varies in position, characterized in that the sensor (20) is constructed as a motion sensor which reacts to the rotation of a running wheel during operation of the suction nozzle or which takes the form of a mercury switch producing continuous contact during movement of the suction nozzle and the sensor is installed in the suction nozzle and drives a multivibrator by means of repeated triggering, this multivibrator keeping the UV radiator (15) switched on until - in the absence of a fresh triggering originating in a direct movement of the suction nozzle - the resting time of the multivibrator expires and the UV radiator is switched off.

2. A suction nozzle in accordance with Claim 1, characterized in that the UV radiator (15) is an elongated tube which is arranged transverse to the normal direction of operating movement of the suction nozzle of the vacuum cleaner and in the process occupies the width of the suction nozzle.

3. A suction nozzle in accordance with Claim 1 or 2, characterized in that the UV radiator (15) is arranged in a compartment (16) separate from the remainder of the nozzle.

4. A suction nozzle in accordance with Claim 3, characterized in that the compartment (16) is covered by a protective cover pervious to the germ-destroying UV radiation.

5. A Suction nozzle in accordance with any one of Claims 1 to 4, characterized in that a reflective material is provided opposite the surface to be treated and behind and/or to the side of the tube of the UV radiator (15), this reflective material reflecting all of the radiation issuing from the UV radiator (15) onto the surface to be treated.

6. A suction nozzle in accordance with Claim 5, characterized in that the reflective material is a coated half shell which is downwardly open in the direction of the surface to be treated, is possibly parabolic in shape, has a reflective inner coating and accommodates the UV radiator.

**Revendications**

1. Buse d'aspiration (10) pour un aspirateur de poussières sous forme d'une buse de sol, une buse de battage de poussières, une buse pour meubles, une buse pour rideaux, pour stériliser des pièces de n'importe quel type ainsi que les objets d'ameublement se trouvant dans les pièces, les rideaux, les revêtements de sol, les tapis ou analogues, avec au moins un projecteur (15) produisant un rayonnement ultraviolet ayant un effet stérilisateur et un détecteur (20) débranchant le projecteur au bout d'un temps prédéfini, buse dans laquelle le rayonnement sortant du projecteur de rayons ultraviolets (15) est dirigé sur la surface traitée par la buse d'aspiration (10), dont la position est variable, buse d'aspiration caractérisée en ce que le détecteur (20) est constitué comme un détecteur de mouvement mis en action par la rotation d'une roue de roulement lors du fonctionnement de la buse d'aspiration ou comme un détecteur de mouvement produisant la forme d'un interrupteur à mercure produisant lors du mouvement de la buse d'aspiration des mises en contact permanentes et est incorporé dans la buse d'aspiration et un organe de basculement est commandé par le détecteur de mouvement grâce à un enclenchement répété, organe qui maintient le projecteur de rayons ultraviolets (15) branché jusqu'à ce que soit écoulé le temps d'arrêt de l'organe de basculement lors de l'absence d'un enclenchement renouvelé revenant à un mouvement direct de la buse d'aspiration et jusqu'à ce que le projecteur de rayons ultraviolets soit débranché.

2. Buse d'aspiration selon la revendication 1, caractérisée en ce que le projecteur de rayons ultraviolets (15) est un tube allongé, qui est disposé transversalement au sens normal du mouvement de travail de la buse d'aspiration de l'aspirateur de poussières en remplissant ce faisant la largeur de la buse d'aspiration.

3. Buse d'aspiration selon la revendication 1 ou 2, caractérisée en ce que le projecteur de rayons ultraviolets (15) est disposé dans un compartiment (16) séparé de la zone résiduelle de la buse.

4. Buse d'aspiration selon la revendication 3, caractérisée en ce que le compartiment (16) est recouvert par un couvercle de protection perméable aux rayons ultraviolets stérilisants.

5. Buse d'aspiration selon l'une des revendications 1 à 4, caractérisée en ce qu'il est prévu une matière réfléchissante disposée vis-à-vis de la surface à traiter et en arrière ou latéralement par rapport au tube du projecteur de rayons ultraviolets (15), matière qui réfléchit la totalité du rayonnement sortant du projecteur de rayons ultraviolets (15) sur la surface à traiter.

6. Buse d'aspiration selon la revendication 5, caractérisée en ce que la matière réfléchissante est une demi-coquille revêtue, ouverte en direction du bas sur la surface à traiter, présentant le cas échéant une forme parabolique avec une couche interne réfléchissante, qui reçoit le rayonnement ultraviolet.

EP 0 327 935 B1

# Fig.1

# Fig.2